(19) Europäisches Patentamt
European Patent Office
Office européen des brevets

(11) **EP 4 455 708 A1**

(12) # EUROPEAN PATENT APPLICATION

(43) Date of publication:
**30.10.2024 Bulletin 2024/44**

(21) Application number: **24172514.2**

(22) Date of filing: **25.04.2024**

(51) International Patent Classification (IPC):
**G01R 33/563** $^{(2006.01)}$ **G01R 33/54** $^{(2006.01)}$

(52) Cooperative Patent Classification (CPC):
**G01R 33/56341; G01R 33/56358;** G01R 33/546

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **25.04.2023 JP 2023071333**

(71) Applicant: **Canon Medical Systems Corporation
Otawara-shi, Tochigi 324-0036 (JP)**

(72) Inventors:
• **KUSAHARA, Hiroshi**
  **Otawara--shi, 324-0036 (JP)**
• **OZAKI, Masanori**
  **Otawara-shi, 324-0036 (JP)**
• **MORI, Takaya**
  **Otawara-shi, 324-0036 (JP)**

(74) Representative: **Marks & Clerk LLP
15 Fetter Lane
London EC4A 1BW (GB)**

(54) **DIFFUSION WEIGHTED MAGNETIC RESONANCE IMAGING APPARATUS AND METHOD FOR VIRTUAL ELASTOGRAPHY**

(57) A magnetic resonance imaging apparatus (100) according to an embodiment includes an obtaining unit (17a) and a calculating unit (17b). The obtaining unit (17a) configured to obtain a plurality of diffusion weighted images including a plurality of first diffusion weighted images taken while a first Motion Probing Gradient (MPG) is applied in a plurality of directions and a plurality of second diffusion weighted images taken while a second MPG having a b value of a different magnitude from that of a b value calculated for the first MPG is applied in the plurality of directions. The calculating unit (17b) configured to calculate an elastic modulus of a biological tissue with respect to each of a plurality of directions, on a basis of the plurality of diffusion weighted images.

FIG.1

**Description**

FIELD

Embodiments described herein relate generally to a magnetic resonance imaging apparatus and a magnetic resonance imaging method.

BACKGROUND

Techniques conventionally known for providing information about firmness of a biological tissue by using a Magnetic Resonance Imaging (MRI) apparatus include, for example, a technique called Magnetic Resonance Elastography (MRE) which utilizes external mechanical vibration and a technique called virtual Magnetic Resonance Elastography (vMRE) which uses diffusion imaging.

However, both of these techniques use calculations assuming isotropy, while anisotropy is not taken into consideration.

SUMMARY OF INVENTION

A magnetic resonance imaging apparatus according to an aspect of the present invention includes:

an obtaining unit configured to obtain a plurality of diffusion weighted images including a plurality of first diffusion weighted images taken while a first Motion Probing Gradient (MPG) is applied in a plurality of directions and a plurality of second diffusion weighted images taken while a second MPG having a b value of a different magnitude from that of a b value calculated for the first MPG is applied in the plurality of directions; and
a calculating unit configured to calculate an elastic modulus of a biological tissue with respect to each of a plurality of directions, on the basis of the plurality of diffusion weighted images.

The calculating unit may be configured to calculate, on the basis of the plurality of diffusion weighted images, a plurality of eigenvalues, by calculating and diagonalizing matrix components of a diffusion tensor, and to calculate, with respect to each of directions corresponding to the plurality of eigenvalues, the elastic modulus for the direction from the eigenvalue.

The calculating unit may be configured to calculate, on the basis of the plurality of diffusion weighted images, a shifted Apparent Diffusion Coefficient (sADC) with respect to each of the plurality of directions and to calculate the elastic modulus from the sADC with respect to each of the directions.

The calculating unit may be configured to calculate, on the basis of the plurality of diffusion weighted images, an attenuation rate of a signal intensity with respect to each of the plurality of directions and to calculate the elastic modulus from the attenuation rate with respect to each of the directions.

The calculating unit may be configured to perform the calculation by applying weights to the elastic moduli in correspondence with the directions.

The calculating unit may be configured to perform the calculation by applying weights to the elastic moduli in correspondence with specific tissues included in the biological tissue.

The magnetic resonance imaging apparatus may further include a display controlling unit configured to display, on the basis of the elastic modulus calculated with respect to each of the directions, information indicating magnitudes and anisotropy of the elastic moduli.

The display controlling unit may be configured to display information indicating, at the same time, both the magnitudes and the anisotropy of the elastic moduli.

The display controlling unit may be configured to display a color map in which magnitudes of the elastic moduli are expressed with darkness levels of a color.

The display controlling unit may be configured to display a color map in which the anisotropy of the elastic moduli is expressed by using different colors.

The display controlling unit may be configured to display information about the elastic moduli calculated in correspondence with the directions, so as to be superimposed together in a single image.

The display controlling unit may be configured to switch in accordance with an instruction from an operator, display between information indicating both the magnitudes and the anisotropy of the elastic moduli at the same time and information indicating only one of the magnitudes and the anisotropy of the elastic moduli.

The display controlling unit may be configured to segment and display the information indicating the magnitudes and the anisotropy of the elastic moduli, in correspondence with compositions of the biological tissue.

The biological tissue may be one of a muscle fiber, a tendon fiber, and a brain tissue.

A magnetic resonance imaging method according to an aspect of the present invention includes:

a step of obtaining a plurality of diffusion weighted images including a plurality of first diffusion weighted images taken while a first Motion Probing Gradient (MPG) is applied in a plurality of directions and a plurality of second diffusion weighted images taken while a second MPG having a b value of a different magnitude from that of a b value calculated for the first MPG is applied in the plurality of directions; and
a step of calculating an elastic modulus of a biological tissue with respect to each of a plurality of directions, on the basis of the plurality of diffusion weighted images.

BRIEF DESCRIPTION OF THE DRAWINGS

FIG. 1 is a diagram illustrating an exemplary configuration of an MRI apparatus according to an embodiment of the present disclosure;
FIG. 2 is a drawing illustrating examples of Diffusion Weighted Images (DWIs) obtained by an obtaining function according to the present embodiment;
FIG. 3 is a drawing illustrating examples of maps of eigenvalues generated by a calculating function according to the present embodiment;
FIG. 4 is a drawing illustrating examples of maps of shifted Apparent Diffusion Coefficients (sADCs) generated by the calculating function according to the present embodiment;
FIG. 5 is a drawing illustrating an example of an information display realized by a display controlling function according to the present embodiment;
FIG. 6 is a drawing illustrating another example of the information display realized by the display controlling function according to the present embodiment; and
FIG. 7 is a flowchart illustrating a processing procedure in processes performed by the MRI apparatus according to the present embodiment.

DETAILED DESCRIPTION

An MRI apparatus according to an embodiment includes an obtaining unit and a calculating unit. The obtaining unit is configured to obtain a plurality of diffusion weighted images including a plurality of first diffusion weighted images taken while a first Motion Probing Gradient (MPG) is applied in a plurality of directions and a plurality of second diffusion weighted images taken while a second MPG having a b value of a different magnitude from that of a b value calculated for the first MPG is applied in the plurality of directions. The calculating unit is configured to calculate an elastic modulus of a biological tissue with respect to each of a plurality of directions, on the basis of the plurality of diffusion weighted images.

Exemplary embodiments of an MRI apparatus and an MRI method according to the present disclosure will be explained in detail below, with reference to the accompanying drawings. Embodiments

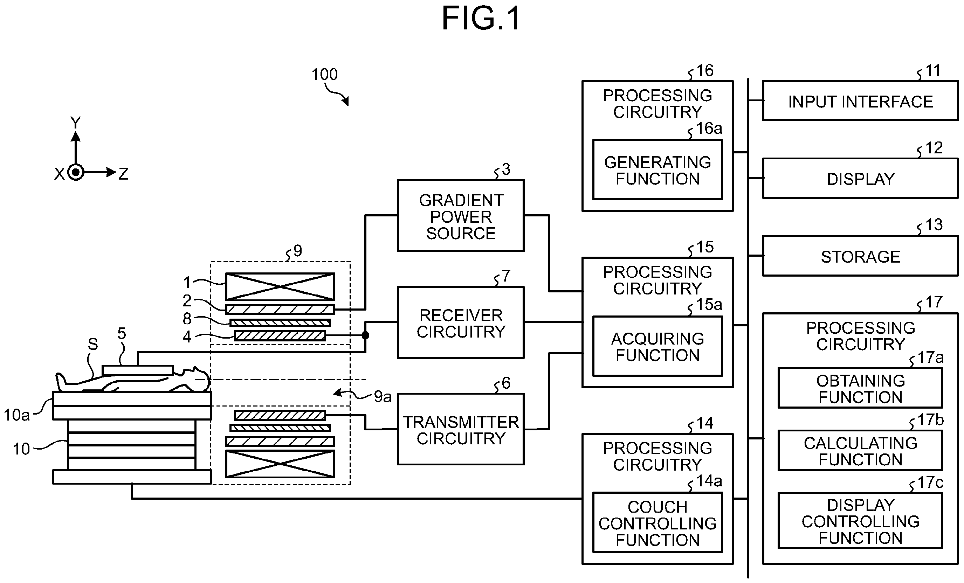

FIG. 1 is a diagram illustrating an exemplary configuration of an MRI apparatus according to an embodiment of the present disclosure.

For example, as illustrated in FIG. 1, an MRI apparatus 100 includes a static magnetic field magnet 1, a gradient coil 2, a gradient power source 3, a whole body Radio Frequency (RF) coil 4, a local RF coil 5, transmitter circuitry 6, receiver circuitry 7, an RF shield 8, a gantry 9, a couch 10, an input interface 11, a display 12, a storage 13, and pieces of processing circuitry 14 to 17.

The static magnetic field magnet 1 is configured to generate a static magnetic field in an imaging space in which an examined subject (hereinafter "patient") S is placed. More specifically, the static magnetic field magnet 1 is formed to have a hollow and substantially circular cylindrical shape (which may have an oval cross-section orthogonal to the central axis thereof) and is configured to generate the static magnetic field in the imaging space formed on the inner circumferential side thereof. For example, the static magnetic field magnet 1 may be a superconductive magnet, a permanent magnet, or the like. In the present example, the superconductive magnet may be structured by using, for instance, a container filled with a cooling agent such as liquid helium and a superconductive coil immersed in the container.

The gradient coil 2 is arranged on the inside of the static magnetic field magnet 1 and is configured to generate gradient magnetic fields in the imaging space in which the patient S is placed. More specifically, the gradient coil 2 is formed to have a hollow and substantially circular cylindrical shape (which may have an oval cross-section orthogonal to the central axis thereof) and includes an X coil, a Y coil, and a Z coil respectively corresponding to an X-axis, a Y-axis, and a Z-axis that are orthogonal to one another. The X coil, the Y coil, and the Z coil are configured to generate, in the imaging space, the gradient magnetic fields that linearly change along the respective axial directions, on the basis of electric currents supplied thereto from the gradient power source 3. In this situation, the Z-axis is set along a magnetic flux in the static magnetic field generated by the static magnetic field magnet 1. Further, the X-axis is set along a horizontal direction orthogonal to the Z-axis. The Y-axis is set along a vertical direction orthogonal to the Z-axis. In this situation, the X-axis, the Y-axis, and the Z-axis structure an apparatus coordinate system unique to the MRI apparatus 100.

The gradient power source 3 is configured to cause, by supplying the electric currents to the gradient coil 2, the gradient magnetic fields to be generated in the imaging space. More specifically, by individually supplying the electric current to each of the X, Y, and Z coils in the gradient coil 2, the gradient power source 3 is configured to cause the gradient magnetic fields to be generated in the imaging space, so as to linearly change along a readout direction, a phase encoding direction, and a slice direction, respectively, that are orthogonal to one another. In this situation, the axis extending along the readout direction, the axis extending along the phase encoding direction, and the axis extending along the slice direction structure a logical coordinate system used for defining slice regions or a volume region subject to the imaging.

More specifically, as each being superimposed on the static magnetic field generated by the static magnetic field magnet 1, the gradient magnetic fields generated along the readout direction, the phase encoding direction, and the slice direction append spatial position information to Nuclear Magnetic Resonance (NMR) signals emitted from the patient S. More specifically, the gradient magnetic field along the readout direction appends position information along the readout direction to the NMR signal, by changing the frequency of the NMR signal in accordance with positions in the readout direction. Further, the gradient magnetic field along the phase encoding direction appends position information along the phase encoding direction to the NMR signal, by changing the phase of the NMR signal in accordance with positions in the phase encoding direction. In addition, when two-dimensional MR images (slice images) are to be taken, the gradient magnetic field along the slice direction determines the positions, the thicknesses, and the quantity of the slices to be imaged, by changing the frequency of the NMR signal in accordance with positions in the slice direction. Further, when a three-dimensional MR image (a volume image) is to be taken, the gradient magnetic field along the slice direction appends position information along the slice direction to the NMR signal, by changing the phase of the NMR signal in accordance with positions in the slice direction.

The whole body RF coil 4 is arranged on the inner circumferential side of the gradient coil 2 and is configured to apply an RF pulse (an excitation pulse or the like) to the patient S placed in the imaging space and to receive the NMR signal (an echo signal or the like) emitted from the patient S due to influence of the RF pulse. More specifically, the whole body RF coil 4 is formed to have a hollow and substantially circular cylindrical shape (which may have an oval cross-section orthogonal to the central axis thereof) and is configured to apply the RF pulse to the patient S placed in the imaging space positioned on the inner circumferential side thereof, on the basis of an RF pulse signal supplied thereto from the transmitter circuitry 6. Further, the whole body RF coil 4 is configured to receive the NMR signal emitted from the patient S due to the influence of the RF pulse and to output the received NMR signal to the receiver circuitry 7. For example, the whole body RF coil 4 may be a birdcage coil or a Transverse Electromagnetic (TEM) coil.

The local RF coil 5 is arranged in the vicinity of the patient S at the time of imaging and is configured to receive the NMR signal emitted from the patient S. More specifically, the local RF coil 5 is prepared for each site of the patient S. At the time of imaging the patient S, the local RF coil 5 is arranged in the vicinity of the site to be imaged and is configured to receive the NMR signal emitted from the patient S due to the influence of the RF pulse applied by the whole body RF coil 4 and to output the received NMR signal to the receiver circuitry 7. For example, the local RF coil 5 may be a surface coil or a phased array coil structured by combining together a plurality of surface coils as coil elements. In addition, the local RF coil 5 may further have a transmitting function to apply an RF pulse to the patient.

The transmitter circuitry 6 is configured to output the RF pulse signal corresponding to a resonance frequency (a Larmor frequency) unique to targeted atomic nuclei placed in the static magnetic field, to the whole body RF coil 4 or the local RF coil 5. More specifically, the transmitter circuitry 6 includes a pulse generator, an RF generator, a modulator, and an amplifier. The pulse generator is configured to generate a waveform of the RF pulse signal. The RF generator is configured to generate an RF signal having the resonance frequency. The modulator is configured to generate the RF pulse signal by modulating the amplitude of the RF signal generated by the RF generator, with the waveform generated by the pulse generator. The amplifier is configured to amplify the RF pulse signal generated by the modulator and to output the amplified signal to the whole body RF coil 4 or the local RF coil 5.

The receiver circuitry 7 is configured to generate NMR data on the basis of the NMR signal output from the whole body RF coil 4 or the local RF coil 5 and to output the generated NMR data to the processing circuitry 15. More specifically, the receiver circuitry 7 includes a selector, a pre-amplifier, a phase detector, and an Analog/Digital (A/D) converter. The selector is configured to selectively receive an input of the NMR signal output from the whole body RF coil 4 or the local RF coil 5. The pre-amplifier is configured to amplify the NMR signal output from the selector. The phase detector is configured to detect the phase of the NMR signal output from the pre-amplifier. The A/D converter is configured to generate the NMR data by converting an analog signal output from the phase detector into a digital signal and to output the generated NMR data to the processing circuitry 15. In this situation, the processes described as being performed by the receiver circuitry 7 do not all necessarily have to be performed by the receiver circuitry 7. One or more of the processes (e.g., the process performed by the A/D converter) may be performed by the whole body RF coil 4 or the local RF coil 5.

The RF shield 8 is arranged between the gradient coil 2 and the whole body RF coil 4 and is configured to shield the gradient coil 2 from the RF pulse generated by the whole body RF coil 4. More specifically, the RF shield 8 is formed to have a hollow and substantially circular cylindrical shape (which may have an oval cross-section orthogonal to the central axis thereof) and is arranged in the space on the inner circumferential side of the gradient coil 2 so as to cover the outer circumferential surface of the whole body RF coil 4.

The gantry 9 has a hollow bore 9a formed to have a substantially circular cylindrical shape (which may have an oval cross-section orthogonal to the central axis thereof) and houses therein the static magnetic field magnet 1, the gradient coil 2, the whole body RF coil 4, and the RF shield 8. More specifically, the gantry 9 houses these elements therein, while the whole body RF coil 4 is arranged on the outer circumferential side of the bore 9a; the RF shield 8 is arranged on the outer circumferential side of the whole body RF coil 4; the gradient coil 2 is arranged on the outer circumferential side of the RF shield 8; and the static magnetic field magnet 1 is arranged on the outer circumferential side of the gradient coil 2. In this situation, the space inside the bore 9a included in the gantry 9 serves as the imaging space in which the patient S is placed at the time of the imaging.

The couch 10 includes a couchtop 10a on which the patient S is placed. At the time of imaging the patient S, the couchtop 10a on which the patient S is placed is moved into the imaging space. For example, the couch 10 is installed in such a manner that the longitudinal direction of the couchtop 10a extends parallel to the central axis of the static magnetic field magnet 1.

The example has been explained where the MRI apparatus 100 has a so-called tunnel-like structure in which the static magnetic field magnet 1, the gradient coil 2, and the whole body RF coil 4 are each formed to have the substantially circular cylindrical shape; however, possible embodiments are not limited to this example. For instance, the MRI apparatus 100 may have a so-called open structure in which a pair of static magnetic field magnets, a pair of gradient coils, and a pair of RF coils are arranged so as to oppose each other, while the imaging space in which the patient S is placed is interposed therebetween. In the open structure, the space interposed between the pair of static magnetic field magnets, the pair of gradient coils, and the pair of RF coils corresponds to the bore in the tunnel-like structure.

The input interface 11 is configured to receive operations to input various types of instructions and various types of information from an operator. More specifically, the input interface 11 is connected to the processing circuitry 17 and is configured to convert the input operations received from the operator into electrical signals and to output the electrical signals to the processing circuitry 17. For example, the input interface 11 is realized by using a trackball, a switch button, a mouse, a keyboard, a touchpad on which an input operation can be performed by touching an operation surface thereof, a touch screen in which a display screen and a touchpad are integrally formed, contactless input circuitry using an optical sensor, audio input circuitry, and/or the like that are used for setting image taking conditions, a Region Of Interest (ROI), and the like. In the present disclosure, the input interface 11 does not necessarily have to include physical operational component parts such as a mouse, a keyboard, and/or the like. Examples of the input interface 11 include, for instance, electrical signal processing circuitry configured to receive an electrical signal corresponding to an input operation from an external input mechanism provided separately from the apparatus and to output the electrical signal to controlling circuitry.

The display 12 is configured to display various types of information. More specifically, the display 12 is connected to the processing circuitry 17 and is configured to convert data of various types of information sent thereto from the processing circuitry 17 into display-purpose electrical signals and to output the electrical signals. For example, the display 12 is realized by using a liquid crystal monitor, a Cathode Ray Tube (CRT) monitor, a touch panel, or the like.

The storage 13 is configured to store various types of data therein. More specifically, the storage 13 is connected to the processing circuitry 14 to 17 and is configured to store therein various types of data input and output by the pieces of processing circuitry. For example, the storage 13 is realized by using a semiconductor memory element such as a Random Access Memory (RAM) or a flash memory, or a hard disk, an optical disk, or the like.

The processing circuitry 14 includes a couch controlling function 14a. The couch controlling function 14a is configured to control operations of the couch 10 by outputting control-purpose electrical signals to the couch 10. For example, via the input interface 11, the couch controlling function 14a is configured to receive, from the operator, an instruction to move the couchtop 10a in a longitudinal direction, an up-and-down direction, or a left- and-right direction and to bring a moving mechanism of the couchtop 10a included in the couch 10 into operation, so as to move the couchtop 10a according to the received instruction.

The processing circuitry 15 includes an acquiring function 15a. The acquiring function 15a is configured to acquire the NMR data of the patient S by executing various types of pulse sequences. More specifically, the acquiring function 15a is configured to execute the various types of pulse sequences by driving the gradient power source 3, the transmitter circuitry 6, and the receiver circuitry 7, according to sequence execution data output from the processing circuitry 17. In this situation, the sequence execution data is data indicating a pulse sequence and is information defining:

timing with which the electric current is to be supplied by the gradient power source 3 to the gradient coil 2 and the intensity of the electric current to be supplied; timing with which the RF pulse signal is to be supplied by the transmitter circuitry 6 to the whole body RF coil 4 and the intensity of the RF pulse signal to be supplied; timing with which the NMR signal is sampled by the receiver circuitry 7; and the like. Further, the acquiring function 15a is configured to receive the NMR data output from the receiver circuitry 7 as a result of executing the pulse sequence and to further store the received NMR data into the storage 13. At that time, the NMR data stored in the storage 13 is stored as k-space data expressing a two- or three-dimensional k-space, as a result of having appended thereto the position information along the directions of the readout direction, the phase encoding direction, and the slice direction, by the gradient magnetic fields explained above.

The processing circuitry 16 includes a generating function 16a. The generating function 16a is configured to generate an MR image from the NMR data acquired by the acquiring function 15a of the processing circuitry 15. More specifically, under control of the processing circuitry 17, the generating function 16a is configured to generate a two- or three- dimensional MR image by reading, from the storage 13, the NMR data acquired by the acquiring function 15a of the processing circuitry 15 and further performing a reconstruction process such as a Fourier transform on the read NMR data. After that, the generating function 16a is configured to store the generated MR image into the storage 13.

The processing circuitry 17 is configured to control the entirety of the MRI apparatus 100, by controlling constituent elements of the MRI apparatus 100. More specifically, the processing circuitry 17 is configured to cause the display 12 to display a Graphical User Interface (GUI) used for receiving operations to input various types of instructions and various types of information from the operator and configured to control the constituent elements of the MRI apparatus 100 in accordance with the input operations received via the input interface 11. For example, the processing circuitry 17 is configured to receive an input of the image taking conditions from the operator and to set the pulse sequence for acquiring the NMR data of the patient S on the basis of the input image taking conditions. Further, by generating and outputting the sequence execution data that represents the pulse sequence having been set, to the processing circuitry 15, the processing circuitry 17 is configured to cause the acquiring function 15a of the processing circuitry 15 to execute any of the various types of pulse sequences. In addition, for example, by controlling the generating function 16a of the processing circuitry 16, the processing circuitry 17 is configured to cause the MR image to be reconstructed from the k-space data acquired by the processing circuitry 15. Also, in response to a request from the operator, the processing circuitry 17 is configured to read the MR image stored in the storage 13 and to cause the display 12 to display the read MR image.

The exemplary configuration of the MRI apparatus 100 according to the present embodiment has thus been explained. The MRI apparatus 100 according to the present embodiment configured as described above has a function of acquiring and providing information about firmness of a biological tissue related to the patient S.

As techniques for providing information about firmness of biological tissues by using an MRI apparatus in this manner, known examples include a technique called MRE which utilizes external mechanical vibration and a technique called vMRE which uses diffusion imaging (see European Patent Application Laid-open No. 3081955 and Denis Le Bihan, Shintaro Ichikawa, Utaroh Motosugi, "Diffusion and Intravoxel Incoherent Motion MR Imaging-based Virtual Elastography: A Hypothesis-generating Study in the Liver", Radiology, Volume 285, No.2, June 12 2017, for example).

MRE is used for obtaining an MR phase image sensitive to a tissue displacement induced by vibration (of which the amplitude is approximately a number of micrometers), by using a shear wave that is generally generated by using an external mechanical vibrator and propagates inside the tissue and a gradient pulse that vibrates at a frequency similar to the vibration frequency caused by the mechanical vibrator. Further, by processing the MR phase image of the propagating shear wave, the image is converted into a shear modulus or the like indicating the firmness of the tissue.

In contrast, vMRE is used for taking a Diffusion Weighted Image (DWI) by using each of the two key b values such as a Low Key b value (LKb) and a High Key b value (HKb) optimized so as to reflect a Gaussian diffusion and a non-Gaussian diffusion and further calculating a shifted Apparent Diffusion Coefficient (sADC) by using relational expression (1) presented below.

$$sADC = \ln(S_{LKb}/S_{HKb})/(HKb - LKb) \tag{1}$$

where, $S_{LKb}$ denotes the signal intensity of the DWI taken by using the LKb, whereas $S_{HKb}$ denotes the signal intensity of the DWI taken by using the HKb.

Further, in vMRE, from the calculated sADC or, more directly, from an attenuation rate $S_{LKb}/S_{HKb}$ of the signal intensities, a shear modulus $\mu_{diff}$ is calculated by using relational expression (2) presented below.

$$\mu_{diff} = g[\ln(S_{LKb}/S_{HKb})] \tag{2}$$

where g denotes a calibration coefficient.

However, those techniques are both based on calculation methods assuming isotropy, while anisotropy is not taken into consideration. For example, in MRE, only cross-sectional planes parallel to a traveling direction of an elastic wave is imaged, and in the first place, a shear modulus or the like is calculated on the assumption that local displacements are isotropic. Further, vMRE uses the single sADC value calculated from the DWIs taken by using the two key b values, while neither anisotropy nor directionality is taken into consideration.

To cope with the circumstances described above, the MRI apparatus 100 according to the present embodiment is configured to be able to acquire and provide the information about the firmness of a biological tissue that takes anisotropy into consideration.

More specifically, in the present embodiment, the processing circuitry 15 includes the abovementioned acquiring function 15a, whereas the processing circuitry 17 includes an obtaining function 17a, a calculating function 17b, and a display controlling function 17c. In this situation, the obtaining function 17a is an example of the obtaining unit. The calculating function 17b is an example of the calculating unit. The display controlling function 17c is an example of a display controlling unit.

The obtaining function 17a is configured to obtain a plurality of DWIs including a plurality of first DWIs taken by the acquiring function 15a of the processing circuitry 15 while a first MPG is applied in a plurality of directions and a plurality of second DWIs taken by the acquiring function 15a of the processing circuitry 15 while a second MPG having a b value of a different magnitude from that of a b value calculated for the first MPG is applied in the plurality of directions.

More specifically, the obtaining function 17a is configured to take the plurality of DWIs by applying the first MPG in the plurality of directions and to take the plurality of DWIs by applying, in the plurality of directions, the second MPG having the b value larger than the b value calculated for the first MPG, by causing the acquiring function 15a of the processing circuitry 15 to execute a pulse sequence of diffusion imaging. After that, the obtaining function 17a is configured to obtain the plurality of DWIs by reading the taken DWIs from the storage 13. In this situation, the magnitude relationship between the b value of the first MPG and the b value of the second MPG is not limited to the above example. The b value of the first MPG may be larger than the b value of the second MPG.

In an embodiment, the difference between the b values of the first and second MPGs is determined according to the degree of diffusion of the tissue subject to a diagnosing process, so that the difference produces a signal difference on the tissue.

In an embodiment, the plurality of directions in which the first MPG and the second MPG are applied comprises two or more directions which are separated by an equal interval. For example, in a 2D imaging, the plurality of directions comprises two or more directions which are separated by an angle of 360/n degrees, where n denotes the number of the directions and is an integer 2 or more. In an embodiment, the plurality of directions may be all set along one desired direction.

In the present embodiment, the obtaining function 17a is configured to take the plurality of DWIs by using the two key b values such as the LKb and the HKb used in vMRE described above, as a first b value and a second b value, respectively.

In an embodiment, the LKb and the HKb are set so as to reflect a Gaussian diffusion and a non-Gaussian diffusion, respectively. Generally, the range value of the LKb is about 50-200 [s/mm2] and the range value of the HKb is about 500-1500 [s/mm2]. However, the range value of the LKb and the range value of the HKb may be changed according to the tissue subject to a diagnosing process.

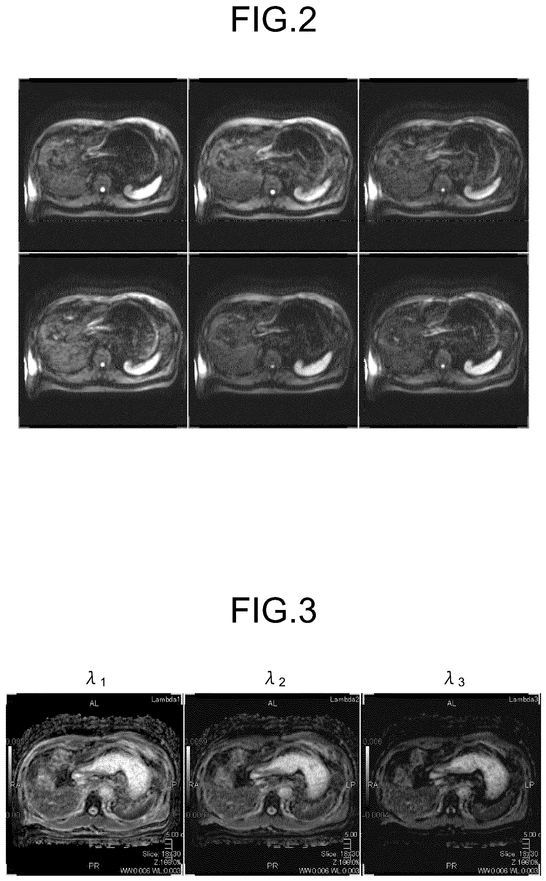

FIG. 2 is a drawing illustrating examples of DWIs obtained by the obtaining function 17a according to the present embodiment.

For example, as illustrated in FIG. 2, the obtaining function 17a is configured to obtain six DWIs taken while the MPG having the LKb is applied in six mutually-different directions. Further, the obtaining function 17a is configured to obtain six DWIs (not illustrated) taken while the MPG having the HKb is applied in the same six directions as the MPG having the LKb.

The present embodiment is explained with the example using the six DWIs taken while the MPG having the LKb is applied in the six directions and the six DWIs taken while the MPG having the HKb is applied in the six directions; however, the quantities of the directions for applying the MPGs and the DWIs are not limited to six and may be four or five or may be seven or more.

Returning to the description of FIG. 1, the calculating function 17b is configured to calculate, on the basis of the plurality of DWIs obtained by the obtaining function 17a, an elastic modulus of a biological tissue with respect to each of the plurality of directions.

In an example, for instance, on the basis of the plurality of DWIs obtained by the obtaining function 17a, the calculating function 17b is configured to calculate a plurality of eigenvalues by calculating and diagonalizing matrix components of a diffusion tensor and to calculate, with respect to each of the directions corresponding to the plurality of eigenvalues, the elastic modulus for the direction from the eigenvalue.

FIG. 3 is a drawing illustrating examples of maps of the eigenvalues generated by the calculating function 17b according to the present embodiment.

For example, on the basis of the six DWIs taken while the MPG having the LKb is applied in the six directions and the six DWIs taken while the MPG having the HKb is applied in the six directions illustrated in FIG. 2, the calculating function 17b is configured to calculate three eigenvalues $\lambda_1$, $\lambda_2$, and $\lambda_3$, by calculating and diagonalizing the components of a diffusion tensor expressed by a matrix of 3 by 3, with respect to each voxel. As a result, for example, maps of the eigenvalues as illustrated in FIG. 3 are generated.

Further, for each voxel, the calculating function 17b is configured to calculate, with respect to each of the directions corresponding to the calculated eigenvalues $\lambda_1$, $\lambda_2$, and $\lambda_3$, an elastic module from the eigenvalue. As a result, maps of elastic modules corresponding to the directions are generated.

In this situation, the calculating function 17b is configured to calculate shear moduli $\mu_{diff1}$, $\mu_{diff2}$, and $\mu_{diff3}$ corresponding to the directions, by using relational expressions (3) and (4) presented below used in MRE described above and substituting the $\lambda$ in the expression with $\lambda1$, $\lambda2$, and $\lambda3$.

$$\lambda = K - 2\mu/3 \qquad\qquad (3)$$

$$E = 2\mu(1 + \sigma) = 3K(1 - 2\sigma) = 9\mu K/3K + \mu \qquad\qquad (4)$$

where E denotes Young's modulus; $\mu$ denotes a shear modulus; $\lambda$ denotes an elastic modulus tensor coefficient; $\sigma$ denotes Poisson's ratio; and K denotes a volume elastic modulus.

Further, in another example, the calculating function 17b may calculate an sADC, for instance, with respect to each of the plurality of directions in which the MPG was applied, on the basis of the plurality of DWIs obtained by the obtaining function 17a, so as to calculate an elastic modulus from the sADC with respect to each of the directions.

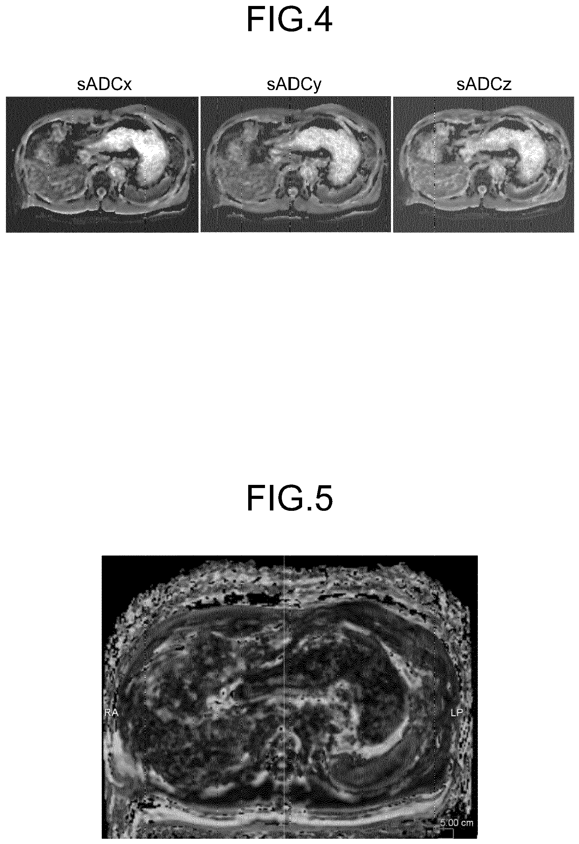

FIG. 4 is a drawing illustrating examples of maps of sADCs generated by the calculating function 17b according to the present embodiment.

For example, on the basis of three DWIs taken while the MPG having the LKB is applied in three directions and three DWIs taken while the MPG having the HKb is applied in three directions, the calculating function 17b is configured to calculate, for each voxel, an sADC with respect to each of the three directions. As a result, as illustrated in FIG. 4, for example, the maps of the sADCs corresponding to the directions are generated. (FIG. 4 illustrates a map of sADCx being sADCs corresponding to an x direction, a map of sADCy being sADCs corresponding to a y direction, and a map of sADCz being sADCs corresponding to a z direction.)

In this situation, by using relational expression (1) (presented below again) used in vMRE described above, the calculating function 17b is configured to calculate the sADCs corresponding to the directions, by substituting, for each direction, $S_{LKb}$ and $S_{HKb}$ in the expression with the signal intensity of the DWI taken by using the LKb and the signal intensity of the DWI taken by using the HKb.

$$sADC = \ln(S_{LKb}/S_{HKb})/(HKb - LKb) \qquad\qquad (1)$$

where $S_{LKb}$ denotes the signal intensity of the DWI taken by using the LKB, whereas $S_{HKb}$ denotes the signal intensity of the DWI taken by using the HKb.

Further, with respect to each of the three directions, the calculating function 17b is configured to calculate, for each voxel, an elastic module from the sADC corresponding to the direction. As a result, the maps of elastic moduli corresponding to the directions are generated.

In this situation, for example, on the basis of the plurality of DWIs obtained by the obtaining function 17a, the calculating function 17b is configured to calculate an attenuation rate of the signal intensity with respect to each of the plurality of directions in which the MPG was applied and to further calculate an elastic modulus from the attenuation rate with respect to each of the directions.

For example, on the basis of three DWIs taken while the MPG having the LKb is applied in three directions and three DWIs taken while the MPG having the HKb is applied in three directions, the calculating function 17b is configured to calculate, for each voxel, the attenuation rate $S_{LKb}/S_{HKb}$ of the signal intensities from the signal intensity $S_{LKb}$ of the DWI taken by using the LKb and the signal intensity $S_{HKb}$ of the DWI taken by using the HKb, with respect to each of the three directions.

After that, the calculating function 17b is configured to calculate, for each voxel, the elastic modulus from the attenuation rate $S_{LKb}/S_{HKb}$ of the signal intensities corresponding to the direction, with respect to each of the three directions. As a result, the maps of the elastic moduli corresponding to the directions are generated.

In this situation, the calculating function 17b is configured to calculate the shear modulus $\mu_{diff}$ corresponding

to each of the directions, by using relational expression (2) (presented below again) used in vMRE described above and substituting, with respect to each of the directions, $S_{LKb}/S_{HKb}$ in the expression with the attenuation rate of the signal intensities.

$$\mu_{diff} = g[\ln(S_{LKb}/S_{HKb})] \qquad\qquad (2)$$

where g denotes the calibration coefficient obtained on the basis of an experiment or experience.

Further, for example, the calculating function 17b may be configured to apply weights to the elastic moduli according to a predetermined standard.

In an example, the calculating function 17b may be configured to perform the calculation by applying the weights to the elastic moduli in correspondence with different directions.

For example, the calculating function 17b may calculate an elastic modulus in a direction parallel to a fiber included in the biological tissue, by applying thereto a weight larger than the weights applied to the elastic moduli in the other directions.

In another example, the calculating function 17b may be configured to perform the calculation by applying the weights to the elastic moduli in correspondence with specific tissues included in the biological tissue.

For example, when the biological tissue is the liver, for the purpose of emphasizing elastic moduli of a liver cirrhosis part as compared to elastic moduli of the other parts, the calculating function 17b may calculate the elastic moduli of the liver cirrhosis part by uniformly applying a larger weight to various directions, as compared to the weights applied to the other parts.

In yet another example, the calculating function 17b may be configured to calculate the elastic moduli, by applying the weights in correspondence with different directions, and also, further applying the weights in correspondence with specific tissues included in the biological tissue.

In this situation, as for methods for applying the weights to the elastic moduli, the calculating function 17b may apply the weights to the eigenvalues or the sADCs in the process of calculating the elastic moduli from the eigenvalues or the sADCs or may calculate the elastic moduli from the eigen values or the sADCs so as to subsequently apply the weights to the calculated elastic moduli.

In other methods for applying the weights to the elastic moduli, the calculating function 17b may apply a weight to a direction or a tissue to be weighted, by multiplying the eigenvalue, the sADC, or the elastic modulus by a weight coefficient of a predetermined magnitude or may apply the weight by enlarging contrast with the other directions or tissues.

Returning to the description of FIG. 1, the display controlling function 17c is configured to display information indicating magnitudes and anisotropy of the elastic moduli, on the basis of the elastic moduli calculated by the calculating function 17b in correspondence with the directions.

In an example, the display controlling function 17c is configured to display, for instance, information indicating both the magnitudes and the anisotropy of the elastic moduli at the same time.

For example, the display controlling function 17c may display information about the elastic moduli calculated by the calculating function 17b in correspondence with the different directions, so as to be superimposed together in a single image.

FIG. 5 is a drawing illustrating an example of the information display realized by the display controlling function 17c according to the present embodiment.

For example, as illustrated in FIG. 5, the display controlling function 17c is configured to generate, on the basis of the elastic moduli calculated by the calculating function 17b in correspondence with the directions, a color map in which, in correspondence with the voxels, the anisotropy of the elastic moduli is expressed by using the colors of RGB, while the magnitudes of the elastic moduli are expressed with darkness levels of the colors, and cause the display 12 to display the color map.

In another example, the display controlling function 17c may be configured, for example, to switch the display between information indicating both the magnitudes and the anisotropy of the elastic moduli at the same time and information indicating only one of the magnitudes and the anisotropy of the elastic moduli, in accordance with an instruction from the operator.

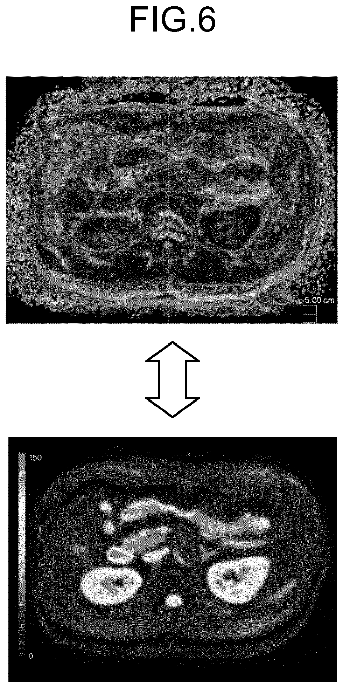

FIG. 6 is a drawing illustrating said another example of the information display realized by the display controlling function 17c according to the present embodiment.

For example, as illustrated in FIG. 6, the display controlling function 17c may be configured to generate a color map (illustrated in the top of the drawing) in which the anisotropy of the elastic moduli is expressed by using the colors of RGB, while the magnitudes of the elastic moduli are expressed with darkness levels of the colors and another color map (illustrated in the bottom of the drawing) in which only the magnitudes of the elastic moduli are expressed by using the colors of RGB, so as to switch the display on the display 12 alternately between the color maps, in accordance with instructions from the operator.

In yet another example, the display controlling function 17c may be configured to switch the display alternately between the color map indicating both the magnitudes and the anisotropy of the elastic moduli and another color map indicating only the anisotropy of the elastic moduli by using the colors of RGB. In yet another example, the display controlling function 17c may be configured to sequentially switch the display, in a predetermined order, among the color map indicating both the magnitudes and the anisotropy of the elastic moduli, the color map indicating only the magnitudes of the elastic moduli, and the color map indicating only the anisotropy of the elastic moduli.

In addition, in yet another example, the display controlling function 17c may be configured, for instance, to segment and display the information indicating the magnitudes and the anisotropy of the elastic moduli, in correspondence with compositions of the biological tissue.

For example, on the basis of the DWIs obtained by the obtaining function 17a, the display controlling function 17c may be configured to divide the biological tissue into a plurality of regions, by segmenting the biological tissue rendered in the images in correspondence with the compositions. Further, for example, among the plurality of divided regions resulting from the segmentation process, the display controlling function 17c may display a color map indicating the magnitudes and the anisotropy of the elastic moduli, only with respect to a region subject to a diagnosing process. For example, among the plurality of divided regions resulting from the segmentation process, the display controlling function 17c may display the color map indicating the magnitudes and the anisotropy of the elastic moduli, only with respect to a region selected by the operator.

The processing functions included in the pieces of processing circuitry 14 to 17 have thus been explained. For example, each of the pieces of processing circuitry 14 to 17 is realized by using a processor. In that situation, the processing functions included in the pieces of processing circuitry are stored in the storage 13 in the form of computer-executable programs. Further, the pieces of processing circuitry are configured to realize the processing functions corresponding to the programs, by reading and executing the programs from the storage 13. In other words, the pieces of processing circuitry that have read the programs have the processing functions illustrated in FIG. 1.

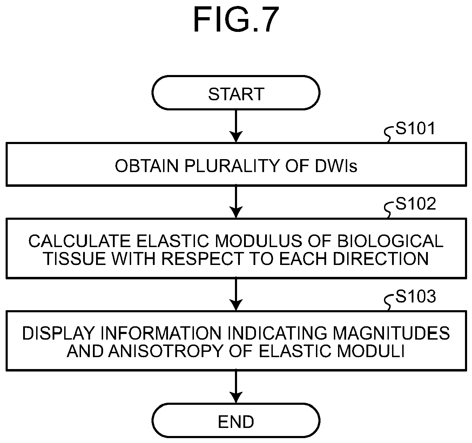

FIG. 7 is a flowchart illustrating a processing procedure in processes performed by the MRI apparatus 100 according to the present embodiment.

For example, as illustrated in FIG. 7, in the present embodiment, to begin with, the obtaining function 17a obtains the plurality of DWIs including the plurality of first DWIs taken while the MPG having the first b value is applied in the plurality of directions and the plurality of second DWIs taken while the MPG having the second b value larger than the first b value is applied in the plurality of directions (step S101). The process at step S101 is realized, for example, as a result of the processing circuitry 17 reading and executing a predetermined program corresponding to the obtaining function 17a from the storage 13.

Subsequently, on the basis of the plurality of DWIs obtained by the obtaining function 17a, the calculating function 17b calculates the elastic modulus of the biological tissue with respect to each of the plurality of directions (step S102). The process at step S102 is realized, for example, as a result of the processing circuitry 17 reading and executing a predetermined program corresponding to the calculating function 17b from the storage 13.

After that, on the basis of the elastic modulus calculated by the calculating function 17b with respect to each of the directions, the display controlling function 17c displays information indicating the magnitudes and the anisotropy of the elastic moduli (step S103). The process at step S103 is realized, for example, as a result of the processing circuitry 17 reading and executing a predetermined program corresponding to the display controlling function 17c from the storage 13.

Further, although the example was explained above in which the pieces of processing circuitry 14 to 17 are each realized by a single processor, possible embodiments are not limited to this example. For instance, the pieces of processing circuitry may be structured by combining together a plurality of independent processors, so that the processing functions are realized as a result of the processors executing the programs. Further, the processing functions of the pieces of processing circuitry may be realized as being distributed among or integrated into one or more pieces of processing circuitry, as appropriate. Furthermore, although the example was explained above in which the single storage (the storage 13) has stored therein the programs corresponding to the processing functions, possible embodiments are not limited to this example. For instance, it is also acceptable to provide a plurality of storages in a distributed manner in correspondence with the pieces of processing circuitry, so that the pieces of processing circuitry read the corresponding programs from the individual storages.

As explained above, in the present embodiment, the obtaining function 17a is configured to obtain the plurality of DWIs including the plurality of first DWIs taken while the first MPG is applied in the plurality of directions and the plurality of second DWIs taken while the second MPG having the b value of a different magnitude from that of the b value calculated for the first MPG is applied in the plurality of directions. Further, on the basis of the plurality of DWIs obtained by the obtaining function 17a, the calculating function 17b is configured to calculate the elastic modulus of the biological tissue with respect to each of the plurality of directions.

Consequently, according to the present embodiment, it is possible to provide the information about the firmness of the biological tissue that takes the anisotropy into consideration.

Further, in the present embodiment, the display controlling function 17c is configured to display the information indicating the magnitudes and the anisotropy of the elastic moduli, on the basis of the elastic modulus calculated by the calculating function 17b with respect to each of the directions.

Consequently, according to the present embodiment, the operator is able to easily understand the relationship between the firmness of the biological tissue and the anisotropy.

In the present example, the biological tissue handled in the present embodiment is, for instance, a biological tissue involving anisotropy such as a muscle fiber, a tendon fiber, or a brain tissue. For instance, examples of the muscle fiber include muscle fibers in a leg, an arm, the abdomen, the back, the myocardia and the like. Further, examples of the tendon fiber include tendon fibers in the Achilles tendon, the patellar tendon, and the like.

For example, when the biological tissue subject to a diagnosing process is a biological tissue expected not to involve hardly any anisotropy such as the liver, it is considered that MRE or vMRE described above will not pose any problem. However, when a biological tissue involving anisotropy such as a muscle fiber, a tendon fiber, or a brain tissue is subject to a diagnosing process, it is necessary to understand not only the degree of firmness, but also anisotropy of the firmness.

To cope with the circumstances described above, according to the present embodiment, it is possible to provide the information about, in addition to the firmness of the biological tissue, the anisotropy of the firmness. As a result, it is possible to perform the diagnosing process and treatment more appropriately.

Further, for example, because MRE described above uses the external mechanical vibration, there is a risk for complications such as bleeding or infection. Further, MRE may require, in some situations, dedicated hardware for causing the mechanical vibration and software for processing the same, which may incur costs. Further, because MRE involves using a specific MRE sequence, in addition to setting the abovementioned hardware on the examined subject (a patient or the like), the examination period for the patient tends to become long. Furthermore, in MRE, because the amplitude of the shear wave quickly attenuates during the propagation through the tissue, it is difficult to use MRE for a tissue positioned deep in the body of the patient. In addition, for MRE, inversely converting a measured phase shift into a wavelength is not easy and may be affected by a confounding effect.

In contrast, according to the present embodiment, it is possible to provide the information about the firmness and the anisotropy of the biological tissue by using the pulse sequence of general diffusion imaging, without using the external mechanical vibration. Consequently, as compared to MRE, it is possible to shorten the examination period, while preventing the risk for complications and the costs from increasing. In addition, it is possible to apply the present disclosure to a tissue positioned deep in the body of a patient, without being affected by a confounding effect.

Other embodiments

In the embodiments described above, the example was explained in which the present disclosure is applied to the MRI apparatus; however, possible embodiments are not limited to this example. For instance, it is also possible to apply the present disclosure to an image processing apparatus connected to an MRI apparatus via a network. In that situation, for example, processing circuitry included in the image processing apparatus has the obtaining function, the calculating function, and the display controlling function described above. Further, the obtaining function is configured to obtain the plurality of DWIs from the MRI apparatus via the network. The display controlling function is configured to display the information indicating the magnitudes and the anisotropy of the elastic moduli on either a display provided for the image processing apparatus or an image display apparatus connected via the network.

Further, in the above embodiments, the example was explained in which the obtaining unit, the calculating unit, and the display controlling unit in the present disclosure are realized by the obtaining function, the calculating function, and the display controlling function of the processing circuitry, respectively; however, possible embodiments are not limited to this example. For instance, instead of being realized by the obtaining function, the calculating function, and the display controlling function described in the embodiments, the functions of the obtaining unit, the calculating unit, and the display controlling unit of the present disclosure may be realized by hardware alone, software alone, or a combination of hardware and software.

In the description above, the example was explained in which the one or more "processors" read and execute the programs corresponding to the processing functions from the storage; however, possible embodiments are not limited to this example. The term "processor" denotes, for example, a Central Processing Unit (CPU), a Graphics Processing Unit (GPU), or circuitry such as an Application Specific Integrated Circuit (ASIC) or a programmable logic device (e.g., a Simple Programmable Logic Device (SPLD), a Complex Programmable Logic Device (CPLD), or a Field Programmable Gate Array (FPGA)). When the processor is a CPU, for example, the processor is configured to realize the processing functions, by reading and executing the programs saved in the storage. In contrast, when the processor is an ASIC, instead of having the programs saved in the storage, the processing functions are directly incorporated as logic circuitry in the circuitry of the processor. The processors in the present embodiments do not each necessarily need to be structured as a single piece of circuitry. It is also acceptable to structure one processor by combining together a plurality of pieces of independent circuitry, so as to realize the processing functions thereof. Furthermore, it is also acceptable to integrate two or more of the constituent elements illustrated in FIG. 1 into a single processor so as to realize the processing functions thereof.

In that situation, the programs executed by the one or more processors may be provided as being incorporated, in advance, in a Read-Only Memory, a storage, or the like. Those programs may be provided as being recorded on a computer-readable storage medium such as a Compact Disk Read-Only Memory (CD-ROM), a Flexible Disk (FD), a Compact Disk Recordable (CD-R), a Digital Versatile Disk (DVD), or the like, in a file in an installable or executable format for those apparatuses. Further, the programs may be stored in a computer connected to a network such as the Internet so as to be provided or distributed as being downloaded via the network. For example, the programs are structured with modules including the functional units described above. In the actual hardware, as a result of a CPU reading and executing the programs from a storage medium such as a ROM, the modules are loaded into a main storage apparatus and generated in the main storage apparatus.

In addition, in the above embodiments, the constituent elements of the apparatuses illustrated in the drawings are based on functional concepts. Thus, it is not necessarily required to physically configure the constituent elements as indicated in the drawings. In other words, specific modes of distribution and integration of the apparatuses are not limited to those illustrated in the drawings. It is acceptable to functionally or physically distribute or integrate all or a part of the apparatuses in any arbitrary units, depending on various loads and the status of use. Further, all or an arbitrary part of the processing functions performed by the apparatuses may be realized by a CPU and a program analyzed and executed by the CPU or may be realized as hardware using wired logic.

Furthermore, with regard to the processes explained in the embodiments described above, it is acceptable to manually perform all or a part of the processes described as being performed automatically. Conversely, by using a publicly-known method, it is also acceptable to automatically perform all or a part of the processes described as being performed manually. Further, unless noted otherwise, it is acceptable to arbitrarily modify any of the processing procedures, the controlling procedures, specific names, and various information including various types of data and parameters that are presented in the above text and the drawings.

According to at least one aspect of the embodiments described above, it is possible to provide the information about the firmness of biological tissues that takes the anisotropy into consideration.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. A magnetic resonance imaging apparatus (100) comprising:

   an obtaining unit (17a) configured to obtain a plurality of diffusion weighted images including a plurality of first diffusion weighted images taken while a first Motion Probing Gradient (MPG) is applied in a plurality of directions and a plurality of second diffusion weighted images taken while a second MPG having a b value of a different magnitude from that of a b value calculated for the first MPG is applied in the plurality of directions; and
   a calculating unit (17b) configured to calculate an elastic modulus of a biological tissue with respect to each of a plurality of directions, on a basis of the plurality of diffusion weighted images.

2. The magnetic resonance imaging apparatus (100) according to claim 1, wherein the calculating unit (17b) is configured to calculate, on the basis of the plurality of diffusion weighted images, a plurality of eigenvalues, by calculating and diagonalizing matrix components of a diffusion tensor, and to calculate, with respect to each of directions corresponding to the plurality of eigenvalues, the elastic modulus for the direction from the eigenvalue.

3. The magnetic resonance imaging apparatus (100) according to claim 1, wherein the calculating unit (17b) is configured to calculate, on the basis of the plurality of diffusion weighted images, a shifted Apparent Diffusion Coefficient (sADC) with respect to each of the plurality of directions and to calculate the elastic modulus from the sADC with respect to each of the directions.

4. The magnetic resonance imaging apparatus (100) according to claim 1, wherein the calculating unit (17b) is configured to calculate, on the basis of the plurality of diffusion weighted images, an attenuation rate of a signal intensity with respect to each of the plurality of directions and to calculate the elastic modulus from the attenuation rate with respect to each of the directions.

5. The magnetic resonance imaging apparatus (100) according to any preceding claim, wherein the calculating unit (17b) is configured to perform the calculation by applying weights to the elastic moduli in correspondence with the directions.

6. The magnetic resonance imaging apparatus (100) according to any preceding claim, wherein the calculating unit (17b) is configured to perform the calculation by applying weights to the elastic moduli in correspondence with specific tissues included in the biological tissue.

7. The magnetic resonance imaging apparatus (100) according to any preceding claim, further comprising a display controlling unit (17c) configured to further display, on a basis of the elastic modulus calculated with respect to each of the directions, information indicating magnitudes and anisotropy of the elastic moduli.

8. The magnetic resonance imaging apparatus (100) according to claim 7, wherein the display controlling unit (17c) is configured to display information indicating, at a same time, both the magnitudes and the anisotropy of the elastic moduli.

9. The magnetic resonance imaging apparatus (100) according to claim 7, wherein the display controlling unit (17c) is configured to display a color map in which magnitudes of the elastic moduli are expressed with darkness levels of a color.

10. The magnetic resonance imaging apparatus (100) according to claim 7, wherein the display controlling unit (17c) is configured to display a color map in which the anisotropy of the elastic moduli is expressed by using different colors.

11. The magnetic resonance imaging apparatus (100) according to claim 7, the display controlling unit (17c) is configured to display information about the elastic moduli calculated in correspondence with the directions, so as to be superimposed together in a single image.

12. The magnetic resonance imaging apparatus (100) according to claim 7, wherein the display controlling unit (17c) is configured to switch, in accordance with an instruction from an operator, display between information indicating both the magnitudes and the anisotropy of the elastic moduli at a same time and information indicating only one of the magnitudes and the anisotropy of the elastic moduli.

13. The magnetic resonance imaging apparatus (100) according to claim 7, wherein the display controlling unit (17c) is configured to segment and display the information indicating the magnitudes and the anisotropy of the elastic moduli, in correspondence with compositions of the biological tissue.

14. The magnetic resonance imaging apparatus (100) according to any one of claims 1 to 12, wherein the biological tissue is one of a muscle fiber, a tendon fiber, and a brain tissue.

15. A magnetic resonance imaging method comprising:

a step (S101) of obtaining a plurality of diffusion weighted images including a plurality of first diffusion weighted images taken while a first MPG is applied in a plurality of directions and a plurality of second diffusion weighted images taken while a second MPG having a b value of a different magnitude from that of a b value calculated for the first MPG is applied in the plurality of directions; and
a step (S102) of calculating an elastic modulus of a biological tissue with respect to each of a plurality of directions, on a basis of the plurality of diffusion weighted images.

# FIG.1

EP 4 455 708 A1

# FIG.2

# FIG.3

$\lambda_1$  $\lambda_2$  $\lambda_3$

# FIG.4

sADCx  sADCy  sADCz

# FIG.5

# FIG.6

# FIG.7

```
                    START

                                      S101
        OBTAIN PLURALITY OF DWIs

                                      S102
    CALCULATE ELASTIC MODULUS OF BIOLOGICAL
     TISSUE WITH RESPECT TO EACH DIRECTION

                                      S103
   DISPLAY INFORMATION INDICATING MAGNITUDES
      AND ANISOTROPY OF ELASTIC MODULI

                     END
```

Europäisches
Patentamt

European
Patent Office

Office européen
des brevets

# EUROPEAN SEARCH REPORT

Application Number

EP 24 17 2514

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 115 105 049 A (FIRST AFFILIATED HOSPITAL MEDICAL COLLEGE XIAN JIAOTONG UNIV) 27 September 2022 (2022-09-27) * paragraphs [0011] - [0028], [0078] - [0093]; figure 2 * | 1-15 | INV. G01R33/563 ADD. G01R33/54 |
| X | JP 2012 143315 A (HITACHI MEDICAL CORP) 2 August 2012 (2012-08-02) * paragraph [0021] - paragraph [0034]; claim 1; figure 3 * | 1-15 | |
| A,D | EP 3 081 955 A1 (COMMISSARIAT ENERGIE ATOMIQUE [FR]) 19 October 2016 (2016-10-19) * the whole document * | 1-15 | |

**TECHNICAL FIELDS SEARCHED (IPC)**

G01R

The present search report has been drawn up for all claims

| Place of search | Date of completion of the search | Examiner |
|---|---|---|
| Munich | 10 September 2024 | Raguin, Guy |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
...............................................................................
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 17 2514

This annex lists the patent family members relating to the patent documents cited in the above-mentioned European search report.
The members are as contained in the European Patent Office EDP file on
The European Patent Office is in no way liable for these particulars which are merely given for the purpose of information.

10-09-2024

| Patent document cited in search report | | Publication date | Patent family member(s) | | Publication date |
|---|---|---|---|---|---|
| CN 115105049 | A | 27-09-2022 | NONE | | |
| JP 2012143315 | A | 02-08-2012 | NONE | | |
| EP 3081955 | A1 | 19-10-2016 | EP | 3081955 A1 | 19-10-2016 |
| | | | EP | 3283896 A1 | 21-02-2018 |
| | | | JP | 6862353 B2 | 21-04-2021 |
| | | | JP | 2018512238 A | 17-05-2018 |
| | | | US | 2018045802 A1 | 15-02-2018 |
| | | | WO | 2016166115 A1 | 20-10-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3081955 A

**Non-patent literature cited in the description**

- **DENIS LE BIHAN ; SHINTARO ICHIKAWA ; UTAR-OH MOTOSUGI.** Diffusion and Intravoxel Incoherent Motion MR Imaging-based Virtual Elastography: A Hypothesis-generating Study in the Liver. *Radiology,* 12 June 2017, vol. 285 (2